# EUROPEAN PATENT APPLICATION

(11) **EP 4 620 387 A1**
(43) Date of publication of application: **24.09.2025**
(21) Application number: 23941082.2
(22) Date of filing: 16.06.2023
(51) Int. Cl.: A61B 5/03, A61B 5/00, A61M 3/02, A61M 1/00

(54) **PRESSURE MEASURING DEVICE, SHEATH HAVING SAME AND PERFUSION ASPIRATION SYSTEM**

(71) Applicant: Zhejiang Yigao Medical Technology Co., Ltd., Hangzhou, Zhejiang 311200 (CN)
(72) Inventor: LI, Fangbing, Hangzhou, Zhejiang 311220 (CN); XU, Penghong, Hangzhou, Zhejiang 311220 (CN); FENG, Donggang, Hangzhou, Zhejiang 311220 (CN); CEN, Jinhua, Hangzhou, Zhejiang 311220 (CN)
(74) Representative: Sun, Yiming
(86) International application number: PCT/CN2023/100687
(87) International publication number: WO 2024/254848

(57) **Abstract**

The present disclosure a pressure detection device, a sheath tube and a perfusion and suction system. The pressure detection device comprises a housing (2) and a pressure detection assembly (5) . the housing (2) has a piezometric cavity (21) and a mounting cavity (20) , the piezometric cavity (21) has an air inlet (22) for communicating with a piezometric interface of the sheath tube and a vent hole (23) for communicating with the outside, and a switch is arranged at the vent hole (23) ; and the pressure detection assembly (5) is arranged in the mounting cavity and includes a gauge pressure sensor, a voltage regulating circuit, an operational amplifier circuit and a conversion circuit; the gauge pressure sensor, the voltage regulating circuit and the operational amplifier circuit are electrically connected with the conversion circuit, and the gauge pressure sensor is used for converting a pressure signal in the piezometric cavity (21) into a voltage signal.

## Description

### TECHNICAL FIELD

The disclosure relates to the field of sheath tubes, in particular to a sheath tube unit capable of measuring pressure and a micro-pressure sensor for a sheath tube.

### BACKGROUND

In a conventional ureteral lithotripsy with flexible endoscopy, the powder of stones and hematuria in renal pelvis can lead to blurred vision, which requires perfusion solution to maintain clear vision. However, at the same time, the intrapelvic pressure will increase significantly due to rapid perfusion and poor return, causing infected urine, bacteria and endotoxin to enter the blood and lymphatic circulation, resulting in postoperative fever, systemic inflammatory response syndrome and even fatal urogenous sepsis in patients. In order to prevent severe infection caused by excessive pressure in the renal pelvis during a flexible contact lenses surgery, it is necessary to control the intraoperative pressure within a safe range. It is further necessary to feedback and adjust the perfusion flow rate and/or negative suction value according to the intraoperative pressure of the renal pelvis. Whether the manometric method used can measure the pressure of the renal pelvis in real time and accurately is the cornerstone to ensure the good performance of the system and the safety of surgical operation. There are three traditional methods for pressure measurement: 1. The first method is to measure the pressure by installing a pressure sensor on the perfusion tubeline, and the normal saline is monitored through silicone film. However, this monitoring feeds back the perfusion pressure, and the monitoring of intrarenal pressure is not the most direct. 2. The second method is to measure the pressure by directly monitoring the intrarenal pressure through a sensor, which is installed in the host controller and connected to an introducer sheath through a tubeline, thus realizing intrarenal pressure monitoring. This kind of monitoring realizes the monitoring and feedback of intrarenal pressure according to the height of liquid level difference in the long tubeline, and the measuring range of the sensor is too large compared with the intrarenal pressure, so the accuracy is not very high. 3. The third method is also to monitor the intrarenal pressure through an external sensor with a small measuring range. It is also connected to the introducer sheath through a tubeline, which is filled with liquid. Pressure monitoring and feedback are realized by perfusing or suctioning liquid. However, this kind of monitoring also faces the problem that if the tubeline is too long, the liquid level will affect the deviation of pressure with the position. In the above three methods, there is a problem that the tubeline from the renal pelvis to the sensor is too long, i.e. the piezometric cavity is too long, which will cause large measurement errors. The most important thing is that the perfusion and suction system has a large volume and many tubes and cables. Doctors need to operate the endoscope, host, suction, perfusion and other operations at the same time, which inevitably will accidentally collide with the piezometric tube, causing sudden changes in pressure and further leading to pressure detection errors. Therefore, there are the above technical problems when the sensor is installed in the perfusion tubeline, the perfusion host or externally. At present, a sensor may be directly built in the distal end of an endoscope or a sheath tube. In this way, the pressure is directly monitored after the endoscope or sheath tube enters the body cavity. The detection error of direct positioning of the renal pelvis will be smaller in this mounting method. However, in an actual operation, the perfusion and suction system is used for laser lithotripsy. Excessive temperature generated during laser lithotripsy will damage tissues, so a perfusion and suction cycle is required to reduce the temperature in the body cavity and maintain constant temperature and pressure. However, the process of laser lithotripsy will cause an instantaneous high pressure, which cannot reflect the real pressure in the cavity. At present, it generally takes 2-5S to display the pressure value collected by the sensor. When the instantaneous high pressure is displayed, the pressure in the cavity has tended to be stable. At this time, the pressure output value cannot truly reflect the real pressure in the cavity and will miscontrol the perfusion and suction unit. Therefore, placing the sensor in the cavity has more disadvantages.

### SUMMARY OF INVENTION

In a first aspect, the present disclosure provides a sheath tube with a pressure measuring function. The sheath tube includes a sheath tube body, the sheath tube body has internally a main channel extending from a proximal end to a distal end and a pressure measuring channel, and an interface is provided at a proximal end of the pressure measuring channel. The sheath tube further includes a pressure detection device, and the pressure detection device includes:
a housing, wherein the housing has a piezometric cavity and a mounting cavity, the piezometric cavity has an air inlet for communicating with a piezometric interface of the sheath tube and a vent hole for communicating with the outside, and a switch is arranged at the vent hole; and
a pressure detection assembly, wherein the pressure detection assembly is arranged in the mounting cavity and includes a gauge pressure sensor, a voltage regulating circuit, an operational amplifier circuit and a conversion circuit; the gauge pressure sensor, the voltage regulating circuit and the operational amplifier circuit are electrically connected with the conversion circuit, and the gauge pressure sensor is used for converting a pressure signal in the piezometric cavity into a voltage signal;
the voltage regulating circuit is configured to increase a voltage difference of the voltage signal, and the operational amplifier circuit multiplies the increased voltage difference; the conversion circuit is used for converting the multiplied voltage signal into a pressure value; and
an aerogel insulator, and the aerogel insulator is used for transmitting a pressure of the piezometric cavity to the pressure detection assembly.

In some embodiments, the gauge pressure sensor has an input voltage positive electrode (+vin), an input voltage negative electrode GND (-vin), an analog signal output voltage positive electrode (+out) and an analog signal output voltage negative electrode (-out); the voltage regulating circuit includes at least a first resistor (R1) and a second resistor (R3); the first resistor (R1) is connected in parallel between the input voltage positive electrode (+vin) and the analog signal output voltage positive electrode (+out), and the second resistor (R3) is connected in parallel between the input voltage negative electrode GND (-vin) and the analog signal output voltage negative electrode (-out).

In some embodiments, the operational amplifier circuit is a GS8332 precision operational amplifier chip, which amplifies the voltage difference by 100 times. **In** some embodiments, the resistance values of the first resistor (R1) and the second resistor (R3) are 100K. In some embodiments, the conversion circuit is a single chip microcomputer stm32f103c8t6.

In some embodiments, the housing includes: a pedestal, wherein a piezometric tube for forming a piezometric cavity is arranged on the pedestal, an air inlet is arranged at one end of the piezometric tube and a vent hole is formed at the other end thereof, and an outlet is arranged on the piezometric tube for outflow of gas in the piezometric tube; an upper cover disposed above the pedestal; a PCB board located inside the pedestal and fixed on the upper cover, the PCB board integrates a pressure detection assembly and a control circuit, and the pressure detection assembly is provided just at the outlet and sealed by an aerogel insulator; a control button disposed on the housing and communicatively connected with the control circuit; and a connection cable connected with the conversion circuit to output a pressure test value.

In a second aspect, the present disclosure provides a detection method for the above-mentioned sheath tube unit. The detection method includes the steps of:
step 1) conversion stage: the gauge pressure sensor converts a pressure signal into µV-level voltage; step 2) voltage difference increasing stage: a first resistor (R1) is connected in parallel between an input voltage positive electrode (+vin) and an analog signal output voltage positive electrode (+out), and a second resistor (R3) is connected in parallel between an input voltage negative electrode GND (-vin) and an analog signal output voltage negative electrode (-out), so as to increase the voltage difference, reach mv level and meet the minimum voltage difference requirement of the operational amplifier circuit; step 3) operational amplifier stage: amplify the voltage difference by 100 times through the operational amplifier circuit to reach V (volt) level in a range (0.5-3.0V); step 4) conversion stage: the voltage difference in step 3) is converted into a pressure value by a conversion circuit for output; and step 5) acquisition stage: the gauge pressure sensor collects for many times, and the single chip microcomputer removes extreme values to calculate for an average and converts it into a pressure value for output.

In a third aspect, the present disclosure provides a pressure detection device, and the pressure detection device is the above-mentioned pressure detection device.

In a fourth aspect, the present disclosure provides an intelligent constant pressure regulating perfusion and suction system, and the system includes
the above-mentioned sheath tube, wherein the sheath tube body further has a suction channel;
an endoscope inserted in a sheath tube, the endoscope having a liquid delivery channel formed therein;
a perfusion unit communicating with the liquid delivery channel for injecting perfusion solution into a body cavity;
a suction unit communicating with a suction channel for pumping out liquid in the human body cavity, the perfusion unit cooperating with the suction unit to maintain the cavity at an appropriate pressure; and
a host controller communicatively connected with the conversion circuit, the perfusion unit and the suction unit, wherein the host controller controls the flow rate and pressure of the perfusion unit and the suction unit according to the pressure value output by the conversion circuit.

In some embodiments, the pressure detection assembly further includes a control circuit, and the control circuit is connected to the host controller for controlling the perfusion unit and the suction unit.

In a fifth aspect, the present disclosure provides an intelligent constant pressure control method for a perfusion and suction system, which includes the following steps:
step 1), preset a highest warning pressure value, a lowest warning pressure value, a pressure control value and a perfusion flow gear; step 2) the host controller controls the feeding of the perfusion solution of the perfusion unit and the suction of the suction unit, and collects the pressure in the cavity and the negative pressure suction pressure; and step 3), a pressure detection device collects the pressure in a cavity and transmits it to the host controller, the host controller actively adjusts perfusion and suction states according to the real-time monitored pressure value and the data change trend of the pressure value, so that the current intracavity pressure is balanced at a pressure control value, thus realizing a balance state between perfusion and suction; the perfusion parameters include a perfusion flow gear, and the suction parameters include an opening threshold of a relief valve and a suction pressure threshold.

The pressure regulation modes of the host controller include a coarse tuning mode, a fine tuning mode and a mixed tuning mode. The coarse tuning mode is in response to an extreme situation where the intracavity pressure is in an extreme condition, and the fine tuning mode is in response to a small pressure difference where the intracavity pressure deviates from the pressure control value; the mixed tuning mode is in response to the intracavity pressure not yet reaching an extreme condition and having a large deviation value.

In some embodiments, when the pressure difference between the intracavity pressure and the pressure control value is in an extreme situation, such as exceeding ±20 mmHg, the coarse tuning mode is activated; when the pressure difference exceeds ±3 mmHg and is within ±8 mmHg, the fine tuning mode is activated; when the detected pressure difference exceeds ±8 mmHg and is within ±20 mmHg, the mixed tuning mode is activated.

In some embodiments, the coarse tuning mode includes the following steps: when the pressure value in the body cavity exceeds the highest warning line, the host controller controls to lower the perfusion flow gear, controls the suction pressure threshold so that the suction flow rate is greater than the perfusion flow rate; when the pressure difference in the cavity exceeds the lowest warning line, the perfusion flow gear will be increased, the relief valve will be opened, and the suction pressure threshold will be tuned to a threshold in a stable state, a rapid balance effect is achieved, so that the intracavity pressure can be free from an extreme state as soon as possible.

In some embodiments, the fine tuning mode includes the steps of maintaining the perfusion gear to maintain operation and keeping the relief valve closed, and performing pressure adjustment by finely tuning the suction pressure threshold.

In some embodiments, when the pressure difference exceeds -3 mmHg and is within -8 mmHg, keep the perfusion flow gear unchanged, keep the relief valve closed, maintain the current operation of perfusion, observe the change trend of uploaded data, increase the suction pressure threshold if it is in an ascending stage, decrease the suction pressure threshold if it is in a descending trend, and the reduction amplitude is greater than the amplitude of increasing the suction pressure threshold in the ascending stage; in this way, in the process of data adjustment, the suction pressure threshold tends to be decreased as a whole to achieve the effect of supercharging by fine tuning; when the pressure difference exceeds 3 mmHg and is within 8 mmHg, keep the perfusion operating at the current level; observe the change trend of uploaded data, increase the "suction pressure threshold" if it is in an ascending stage, decrease the suction pressure threshold if it is in a descending trend, and the reduction amplitude is smaller than the amplitude of increasing the suction pressure threshold in the ascending stage; in the process of data adjustment, the "suction pressure threshold" tends to be increased as a whole to achieve the effect of pressure reduction.

In some embodiments, the mixed tuning mode includes the steps of combining fine tuning of the perfusion flow, fine tuning of the relief valve and fine tuning of the suction pressure threshold to jointly act on the intracavity pressure through jointly tuning the perfusion flow gear, the relief valve and the suction pressure threshold, so as to achieve the effect of supercharging or pressure reduction.

In some embodiments, when the detected pressure difference exceeds 8 mmHg and is within 20 mmHg, appropriately reduce the perfusion flow rate, observe the change trend of uploaded data, increase the suction pressure threshold if it is in an ascending stage, and decrease the suction pressure threshold if it is in a descending state, and the reduction amplitude is smaller than the amplitude of increasing the suction pressure threshold in the ascending stage; in this way, in the process of data adjustment, the "suction pressure threshold" tends to be increased as a whole to achieve the effect of pressure reduction; when the pressure difference is detected lower than -8 mmHg and is within -20 mmHg, keep the perfusion flow rate at the current level, open the relief valve in stages, observe the change trend of uploaded data, increase the suction pressure threshold if it is in an ascending stage, decrease the "suction pressure threshold" if it is in a descending trend, and the reduction amplitude is greater than the amplitude of increasing the suction pressure threshold in the ascending stage; in this way, in the process of data adjustment, the "suction pressure threshold" tends to be decreased as a whole to achieve the effect of supercharging;

In some embodiments, the amplitude of tuning the suction pressure threshold increases with an increase in the pressure difference between the intracavity pressure and the pressure control value.

According to the present disclosure, the pressure detection device is installed at a proximal end of the sheath tube, firstly solving the problem that the traditional pressure detection pipeline is too long and has a high error. Secondly, in an actual operation, the sheath tube is located in the cavity, so doctors will not accidentally touch the sheath tube, thus avoiding the pressure detection error caused by a doctor's accidental collision with the piezometric tube. Finally, according to the present disclosure, the pressure detection device placed at the proximal end of the sheath tube will not detect the instantaneous high pressure caused by laser lithotripsy, and the stable pressure in the cavity can be detected with small detection error and high accuracy. A sheath tube and a pressure detection assembly are combined in the sheath tube unit provided by the present disclosure. Based on the relationship between the voltage and the pressure of the gauge pressure sensor, after passing through the voltage regulating circuit and the operational amplifier circuit, the voltage signal is converted into a specific pressure value through conversion by a single chip microcomputer, so as to realize real-time monitoring of the pressure in the cavity, with high detection accuracy and high sensitivity.

### BRIEF DESCRIPTION OF DRAWINGS

FIG. 1 is a structural schematic diagram of the sheath tube provided by the present disclosure;
FIGS. 2-6 are structural schematic diagrams of the pressure detection device provided by the present disclosure;
FIG. 7 is a schematic diagram for the working principle of the pressure detection device provided by the present disclosure;
FIG. 8 is a structural schematic diagram of the pressure detection assembly provided by the present disclosure;
FIG. 9 is a structural schematic diagram of the sheath tube body provided by the present disclosure;
FIG. 10 is a structural schematic diagram of the perfusion system provided by the present disclosure;
FIG. 11 is a schematic diagram of the gauge pressure sensor circuit provided by the present disclosure;
FIG. 12 is a schematic diagram of the voltage regulating circuit provided by the present disclosure;
FIG. 13 is a schematic diagram of the operational amplifier circuit provided by the present disclosure;
FIGS. 14-16 are process curve diagrams of the control method provided by the present disclosure.

### DESCRIPTION OF EMBODIMENTS

In the description of the present disclosure, it should be understood that the azimuth or positional relationships indicated by terms such as "upper", "lower", "front", "rear", "left", "right", "inside", "outside", "proximal" and "distal" are based on those shown in the drawings, which are only for facilitating the description of the present disclosure and simplifying the description, rather than indicating or implying that the target device or component must have a specific orientation and be structured and operated at a specific orientation. Therefore, it cannot be construed as a limitation of the present disclosure. In addition, the features defined as "first" and "second" may explicitly or implicitly include one or more of such features.

In the description of the present disclosure, it should be noted that unless otherwise explicitly specified and limited, the terms "installation", "connection with" and "connection to" shall be understood as a general sense. For example, they can be fixed connection, removable connection or integral connection. They may be mechanically or electrically connected. It can be directly connected or indirectly connected through intermediate media, and it can be the connection between two elements. Those of ordinary skill in the art can understand the specific meanings of the above terms in the present disclosure as appropriate.

### Embodiment 1

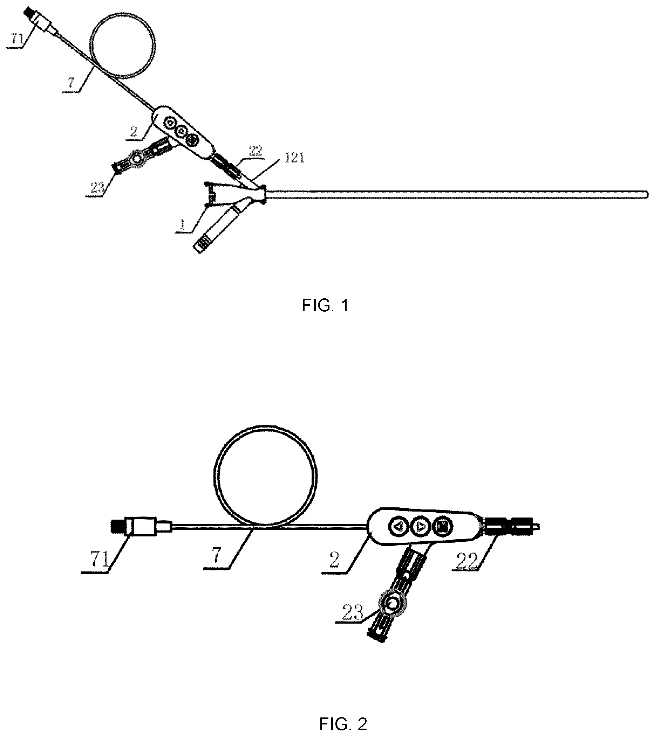

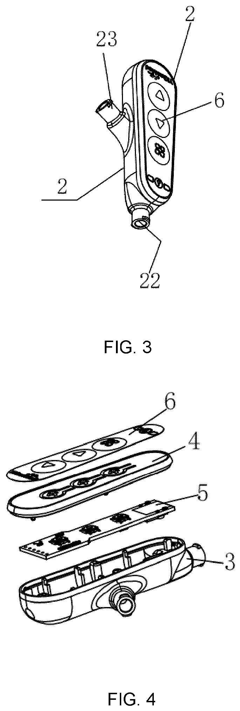

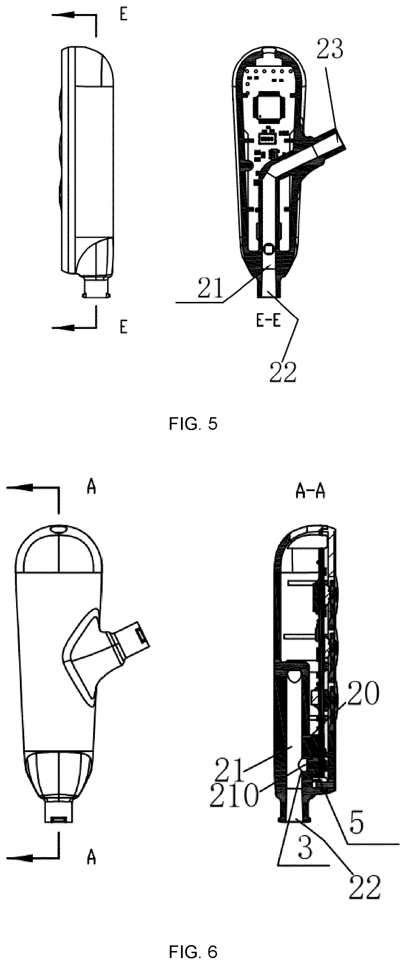

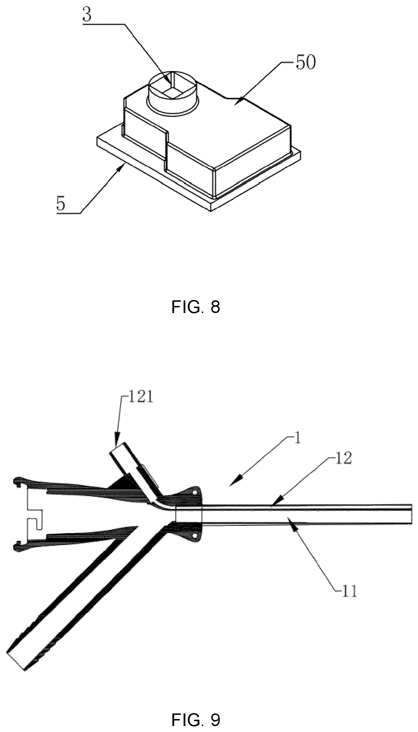

Referring to FIGS. 1-9, the embodiment of the present disclosure provides a sheath tube with a pressure sensing function. The sheath tube includes a sheath tube body 1 and a pressure detection device; the sheath tube body has internally a main channel 11 extending from a proximal end to a distal end and a pressure measuring channel 12. The main channel 11 is used for passing instruments, perfusion solution and suction solution, and an interface 121 is provided at a proximal end of the pressure measuring channel 12; the pressure detection device is assembled at the interface for detecting the pressure in the piezometric cavity. In this way, according to the present disclosure, the pressure detection device is installed at a proximal end of the sheath tube, solving the problem that the traditional pressure detection pipeline is too long and has a high error. In an actual operation, the sheath tube is located in the cavity, so doctors will not accidentally touch the sheath tube, thus avoiding the pressure detection error caused by a doctor's accidental collision with the piezometric tube. In this embodiment, the pressure detection device placed at the proximal end of the sheath tube will not detect the instantaneous high pressure caused by laser lithotripsy, and the stable pressure in the cavity can be detected with small detection error and high accuracy. The current pressure detection device has technical problems such as difficult assembly, inaccurate detection, large error and low sensitivity. Especially when the gauge pressure sensor detects the pressure in the renal pelvis, since the directly monitored pressure is at µV-level voltage, if it is amplified directly by an operational amplifier circuit, the µV-level voltage is difficult to meet the amplification requirements of the operational amplifier circuit, and the voltage difference and pressure should be kept increasing proportionally during the amplification process. Based on this, the pressure detection device provided in this embodiment includes a housing 2, an aerogel insulator 3 and a pressure detection assembly 5. The housing 2 has a piezometric cavity 21 and a mounting cavity 20. The piezometric cavity 21 has an air inlet 22 for communicating with a piezometric interface of the sheath tube and a vent hole 23 for communicating with the outside. A switch is arranged at the vent hole 23. The pressure detection assembly 5 is arranged in the mounting cavity 20, and the piezometric cavity 21 aligns with the pressure detection assembly 5 to form an outlet 210. The outlet 210 is sealed by the aerogel insulator 3, and the aerogel insulator 3 is used for transmitting the pressure of the piezometric cavity 21 to the pressure detection assembly 5.

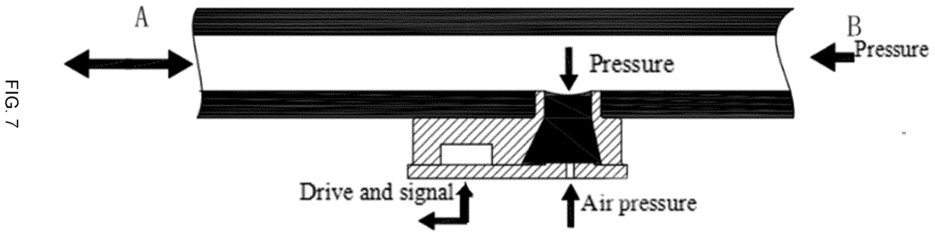

As shown in FIG. 7, the A end of vent hole 23 is connected with a switch, and when it is opened, the pressure detection device can be connected to the atmosphere for zero calibration; when it is closed, the pressure can be measured. The B end of the air inlet 22 is connected to the interface 121 of the sheath tube body; after resetting, turn off the switch at A end. When there is pressure at B end, the pressure will pass through polycarbonate insulating gel and be converted into signal output by the gauge pressure sensor. Turn on the switch to connect the air to the air inlet, and the subsequent measured pressure is tested based on the current pressure. Each sensor has a certain difference. The software is used for calibration, and the calibration data is saved in a single chip microcomputer. The calibration data will still be saved even if it is powered off; therefore, each sensor only needs to be calibrated once.

The pressure detection assembly provided in this embodiment includes a gauge pressure sensor, a voltage regulating circuit, an operational amplifier circuit and a conversion circuit; the gauge pressure sensor, the voltage regulating circuit and the operational amplifier circuit are electrically connected with the conversion circuit, and the gauge pressure sensor is used for converting a pressure signal in the piezometric cavity into a voltage signal; the voltage regulating circuit is configured to increase a voltage difference of the voltage signal, and the operational amplifier circuit multiplies the increased voltage difference; the conversion circuit is used for converting the multiplied voltage signal into a pressure value. In this embodiment, after the process of pressure → voltage, the pressure difference is adjusted by pull-up and pull-down resistors and then amplified. Finally, it is further amplified by an operational amplifier circuit and transmitted to a single chip microcomputer for AD conversion to obtain a specific collected pressure value.

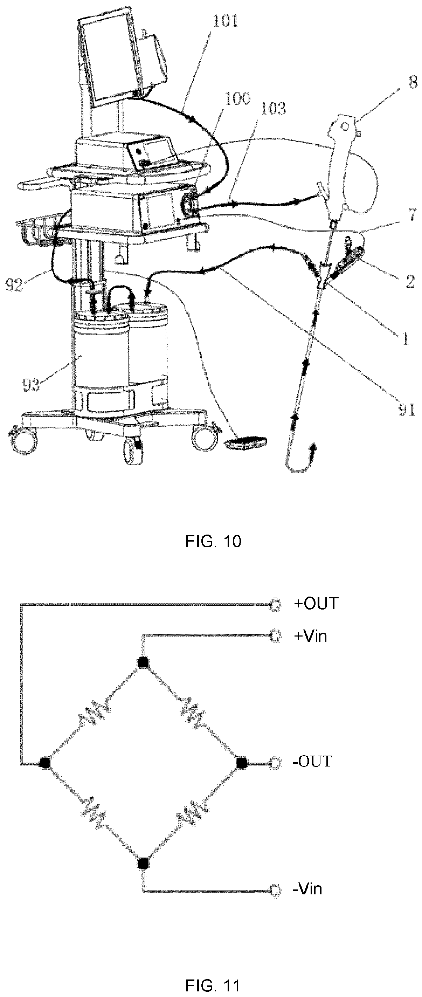

As shown in FIG. 11, this figure shows the detection circuit inside the gauge pressure sensor, and the main function of this part is to convert a pressure signal into a voltage signal with a conversion relationship of 5µV/V/mmHg. If the signal is directly acquired by 12-bit AD conversion, it needs to be amplified because the voltage difference formed by voltage and pressure is too small to be directly amplified. The acquisition accuracy of 12-bit AD of a single chip microcomputer is 3.3V/4096=8.056mV, which is far greater than the relationship between pressure and voltage (5µV/V/mmHg) of this gauge pressure sensor. Therefore, this embodiment increases the voltage difference between the analog signal output positive electrode V+ and the analog signal output negative electrode V-, while maintaining a linear relationship between the voltage and the pressure.

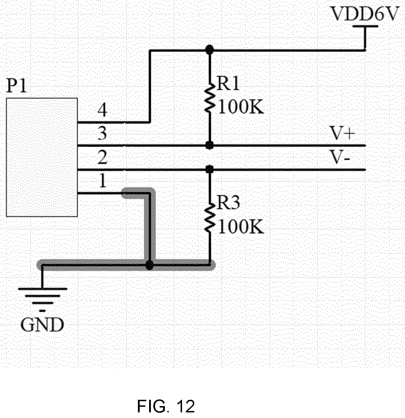

The gauge pressure sensor has an input voltage positive electrode (+vin), an input voltage negative electrode GND (-vin), an analog signal output voltage positive electrode (+out) and an analog signal output voltage negative electrode (-out). As shown in FIG. 12, the voltage regulating circuit includes a first resistor (R1) and a second resistor (R3), wherein the first resistor (R1) is connected in parallel between an input voltage positive electrode (+vin) and an analog signal output voltage positive electrode (+out), R1 is applied between V+ and VDD6V, and the resistor is connected in parallel with a pull-up resistor inside the sensor; after the resistors are connected in parallel, the resistance decreases, so that the voltage of V+ is greater than the voltage of the sensor output V+. The second resistor (R3) is connected in parallel between the input voltage negative electrode GND(-vin) and the analog signal output voltage negative electrode (-out), R3 is added between V- and GND, and is connected in parallel with a pull-down resistor inside the sensor, then the voltage of V- is smaller than that of the sensor. Then, the voltage difference between V+ and V- realized by this part of circuit is increased to prepare for the next step of operational amplifier.

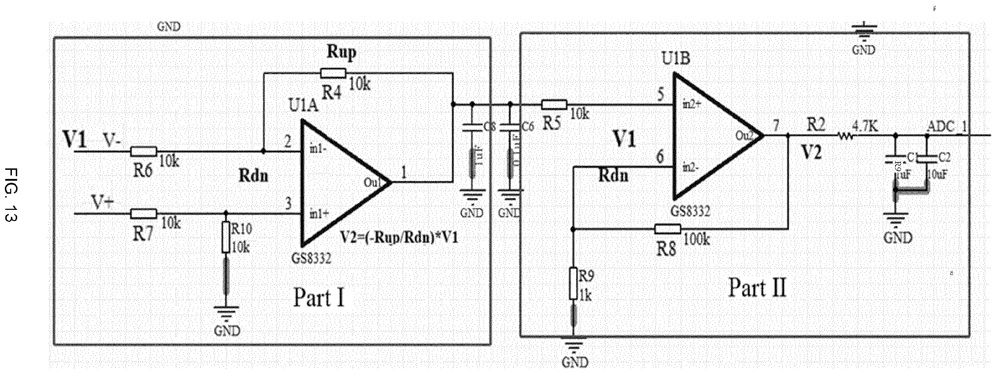

As shown in FIG. 13, in order to maintain the linear relationship between sensor pressure and voltage, a GS8332 precision operational amplifier chip is used to amplify the voltage difference by 100 times.

Optionally, the operational amplifier circuit is a GS8332 precision operational amplifier chip, which amplifies the voltage difference by 100 times. The resistance values of the first resistor (R1) and the second resistor (R3) are 100K. The conversion circuit is a single chip microcomputer stm32f103c8t6.

The detection method of the present disclosure includes the following steps:
step 1) conversion stage: the gauge pressure sensor converts a pressure signal into µV-level voltage;
step 2) voltage difference increasing stage: a first resistor (R1) is connected in parallel between an input voltage positive electrode (+vin) and an analog signal output voltage positive electrode (+out), and a second resistor (R3) is connected in parallel between an input voltage negative electrode GND (-vin) and an analog signal output voltage negative electrode (-out), so as to increase the voltage difference and meet the minimum voltage difference requirement of the operational amplifier circuit;
step 3) operational amplifier stage: amplify the voltage difference by 100 times through the operational amplifier circuit to reach V (volt) level;
step 4) conversion stage: the voltage difference in step 3) is converted into a pressure value by a conversion circuit for output; and
step 5) acquisition stage: the gauge pressure sensor collects for many times, and the single chip microcomputer removes extreme values to calculate for an average and converts it into a pressure value for output.

### Exemplarily,

### Part I:

As shown in FIG. 11, the analog signal output positive electrode V+ and the analog signal output negative electrode V- are connected to R6, R7, R4, R6, R7 and R10, all of which are 10K, and the voltage at C8 is VOUT. According to the operational amplifier formula VOUT-(V+/2)=(V+/2)-(V-), it can be obtained that VOUT = (V+)-(V-). Therefore, this part of function is to calculate the voltage difference between the analog signal output positive electrode V+ and the analog signal output negative electrode V-.

### Part II:

As shown in FIG. 12, R9=1k, R8=100K, the voltage difference between V+ and V- is mainly amplified by these two resistors. According to the relationship between R8 and R9, the amplification factor of the voltage difference is 100 times, and the final output voltage range is 0.5V-3.0V, which meets the AD acquisition range of stm32f103c8t6.

Compared with the prior art, the pressure detection device provided by the present disclosure has the following beneficial effects: 1. Data can be saved after sensor calibration. 2. The calibration accuracy can be adjusted (by controlling the pull-up and pull-down resistors). 3. The analog signal is converted into digital signal, which is sent by a upper computer on a serial port to increase the compatibility of a sensor. 4. The sensor module is independent, which can be used in more scenarios.

In this embodiment, the housing includes a pedestal 3, an upper cover 4, a PCB board 5, a control button 6 and a connecting cable 7; the pedestal 1 is provided with a piezometric tube 21 for forming a piezometric cavity; one end of the piezometric tube 21 is provided with an air inlet 22 and the other end is formed with a vent hole 23. The piezometric tube 21 is provided with an outlet 210 for outflow of gas in the piezometric tube; the upper cover 4 is arranged above the pedestal; the PCB board 5 is located inside the pedestal and fixed on the upper cover; the PCB board integrates a pressure detection assembly and a control circuit, and the pressure detection assembly is provided just at the outlet 210 and sealed by an aerogel insulator 3; the control button is arranged on the housing and communicatively connected with the control circuit; the connection cable 7 is connected with the conversion circuit to output a pressure test value, and the connection cable has an aviation connector 71.

Specifically, the PCB board, the pressure detection assembly and the control circuit are installed in a housing 50. A through-hole is formed on the housing. The through-hole is sealed with polycarbonate insulating gel, and the through-hole is arranged facing the outlet 210.

In some embodiments, the pressure detection assembly further includes a control circuit, and the control circuit is connected to the host controller for controlling the perfusion unit and the suction unit.

The sheath tube unit provided by the present disclosure combines the sheath tube and the pressure detection assembly. Based on the relationship between the voltage and the pressure of the gauge pressure sensor, after passing through the voltage regulating circuit and the operational amplifier circuit, the voltage signal is converted into a specific pressure value through AD conversion, thus avoiding the pressure deviation caused by long tubeline and finally realizing real-time monitoring of the pressure in the cavity.

### Embodiment 2

As shown in FIG. 10, the present embodiment provides a perfusion and suction system under intelligent constant pressure control. The system includes the sheath tube of embodiment 1, an endoscope 8, a perfusion unit, a suction unit and a host controller 100. The sheath tube further has a suction channel, and in this embodiment, the suction channel and the main channel are the same channel; the endoscope 8 is inserted into the sheath tube, and a liquid delivery channel is formed in the endoscope 8; the perfusion unit 10 communicates with the liquid delivery channel for injecting the perfusion solution into the body cavity;

The suction unit is used for suctioning the waste liquid and stones in the cavity through a sheath tube by negative pressure of the host, collecting the waste liquid and stones into a collector through negative pressure suction tubes 2 and the sheath tube 1, and the perfusion unit cooperates with the suction unit to keep the cavity at an appropriate pressure.

The perfusion unit includes a liquid inlet tube 101, a liquid outlet tube 103 and a perfusion pump; the perfusion pump is connected with the liquid storage bag through the liquid inlet tube 101; the perfusion pump is communicated with a liquid delivery channel of the endoscope 8 through the liquid outlet tube 103;

the suction unit includes a suction pump, a first negative pressure suction tube 91, a suction container 93, a second negative pressure suction tube 92 and a pressure sensor; one end of the first negative pressure suction tube 91 is connected with the sheath tube 1 and the other end thereof is connected with the suction container 93; one end of the second negative pressure suction tube 92 is connected with the suction container 93 and the other end thereof is connected with the suction pump; the pressure sensor is used for detecting the intracavity pressure in the suction container 93, and the suction container 93 is provided with a relief valve.

The host controller 100 is communicatively connected with the conversion circuit, the perfusion unit and the suction unit. The host controller controls the flow rate and pressure of the perfusion unit and the suction unit according to the pressure value output by the conversion circuit. In this embodiment, the perfusion pump and the suction pump are integrated with the host controller and installed. Optionally, the perfusion pump is a peristaltic pump, and the suction pump is a diaphragm pump.

The pressure detection device provided in this embodiment is further provided with a control circuit. The control circuit is connected to the host controller 100 through the cable connector 71. The single chip microcomputer transmits the pressure value to the host controller, and the host controller adjusts the flow rate and pressure of the perfusion unit and the suction unit according to the pressure output value so as to adjust the pressure in the cavity.

The system also includes a display, the display is capable of displaying pressure output values.

In one embodiment of the present disclosure, the present disclosure provides an intelligent constant pressure control method, which includes the following:
step 1), a pressure of the pressure detection device is output to a host controller, and the host controller compares the pressure with a preset ideal pressure; if the pressure fails to reach the ideal pressure, the parameters of perfusion and suction, such as pressure, temperature and flow rate, are controlled by the host controller; and
step 2), the pressure detection device is provided with a control button, which can be used to directly control the perfusion unit and the suction unit to regulate the pressure in the body cavity.

In a preferred embodiment of the present disclosure, the suction unit further includes a pressure sensor for detecting the intracavity pressure of the suction container 93, and the suction container 93 is provided with a relief valve.

The host controller controls the change of the suction pressure threshold. When the pressure of the suction container 93 is greater than the suction pressure threshold, the suction pump stops; when the pressure of the suction container is less than the suction pressure threshold and the current cavity pressure is greater than the "pressure control value -3 mmHg", the suction pump is turned on, and the pressure of the suction container is adjusted by changing the suction pressure threshold. Different pressures of the suction containers lead to different suction flow rates. By adjusting the suction flow rate and the perfusion flow rate, a certain pressure in the cavity is maintained to achieve a dynamic balance between perfusion and suction.

The host controller further presets a highest warning pressure value, a lowest warning pressure value, a pressure control value and a perfusion flow gear. Here, the "pressure control value" refers to the ideal pressure value or pressure range in the preset cavity, and the "warning pressure value" here refers to the state that the difference between the intracavity pressure and the pressure control value exceeds this value. The pressure detection device detects the intracavity pressure every 0.25s. When the intracavity pressure is not at the "pressure control value ±8mmHg", it indicates that the pressure is higher or lower than the "pressure control value" at this time, and the pressure difference is too large. At this time, it is necessary to adjust the pressure in a wide range by coarse tuning, such as tuning the perfusion gear and tuning the relief valve to quickly regulate the intracavity pressure close to or reach the pressure control value. However, when the intracavity pressure is at a "pressure control value ±8mmHg", the difference between the intracavity pressure and the pressure control value is small. If it continues to be adjusted by coarse tuning of such as perfusion gear or relief valve, it will easily cause the intracavity pressure to go to the other extreme, making it difficult to reach or approach the control value. In addition, the suction pressure of the suction pump of the suction unit is relatively large, and it is also difficult to achieve the effect of fine tuning if the pressure is directly adjusted by the suction pump. Based on this, the present disclosure finely tunes the pressure threshold value of the suction container; to sum up, the purpose of the present disclosure is to obtain a dynamic balance of intracavity pressure, and maintain the intracavity pressure at a dynamic balance state of the pressure control value through the combination of coarse tuning and fine tuning.

The present disclosure provides a constant pressure control method for a perfusion and suction system, which includes the following steps:
step 1), preset a highest warning pressure value, a lowest warning pressure value, a pressure control value and a perfusion flow gear;
step 2) the host controller controls the feeding of the perfusion solution of the perfusion unit and the suction of the suction unit, and collects the pressure in the cavity and the negative pressure suction pressure; and
step 3), the pressure detection device collects the intracavity pressure and transmits it to a host controller, and the host controller automatically adjusts the corresponding state system according to the real-time monitored pressure value and the data change trend of the pressure values, so that the current intracavity pressure is balanced at the pressure control value; when the data exceeds a standard, the system will automatically adjust its own perfusion and suction status to reach a balanced state of perfusion and suction as soon as possible while maintaining a certain intracavity pressure; the perfusion parameters include a perfusion flow gear, and the suction parameters include an opening threshold of a relief valve and a suction pressure threshold.

The pressure regulation modes of the host controller include a coarse tuning mode, a fine tuning mode and a mixed tuning mode. The coarse tuning mode is in response to an extreme situation where the intracavity pressure is in an extreme condition, and the fine tuning mode is in response to a small pressure difference where the intracavity pressure deviates from the pressure control value. The mixed tuning mode is in response to the intracavity pressure not yet reaching an extreme condition and having a large deviation value. When the pressure difference between the intracavity pressure and the pressure control value is in an extreme situation, such as exceeding ±20 mmHg, the coarse tuning mode is activated; when the pressure difference exceeds ±3 mmHg and is within ±8 mmHg, the fine tuning mode is activated; when the detected pressure difference exceeds ±8 mmHg and is within ±20 mmHg, the mixed tuning mode is activated.

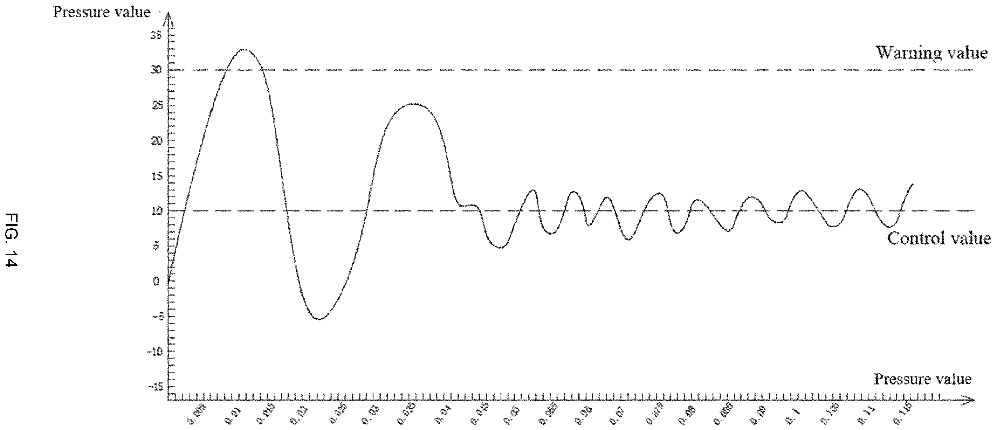

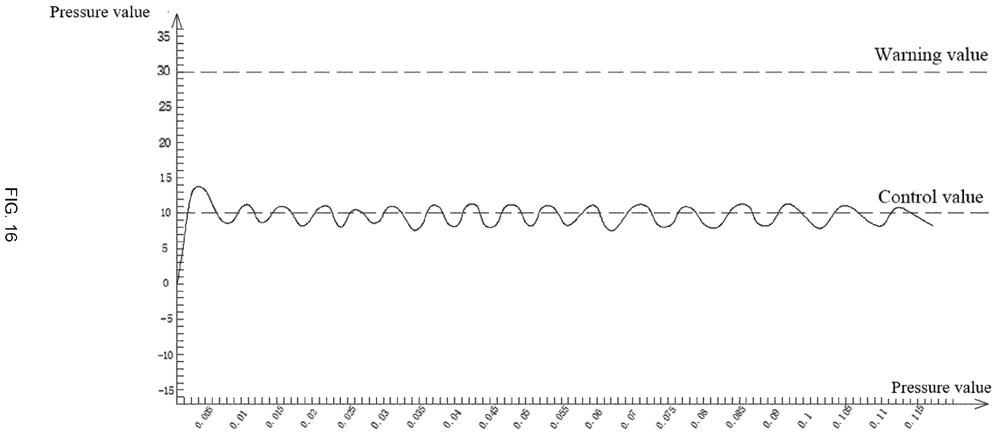

The pressure control value may be a point value or an interval value. In some embodiments of the present disclosure, as shown in FIGS. 14-16, the pressure control value is 10 mmHg, and when the difference between the intracavity pressure and the pressure control value is within ±3 mmHg, the pressure regulation process is started.

As shown in FIG. 14, when it is detected that the intracavity pressure exceeds the highest warning line, i.e. the pressure difference between the intracavity pressure and the pressure control value is in an extreme situation, for example, greater than 20 mmHg, the host controller controls to lower the perfusion flow gear, controls the suction pressure threshold so that the suction flow rate is greater than the perfusion flow rate; when the pressure difference in the cavity exceeds the lowest warning line, the perfusion flow gear is tuned to the maximum, the relief valve will be opened, and the suction pressure threshold will be tuned to a threshold in a stable state, a rapid balance effect is achieved, so that the equipment can be free from the current state as soon as possible.

In this way, the system response is accelerated and the static error is reduced. However, this tuning mode will increase the overshoot and deteriorate the stability. In this embodiment, the perfusion flow gear is adjusted to the lowest gear or the highest gear to speed up the pressure adjustment. At the same time, in order to avoid an excessive adjustment amplitude of the pressure, the suction pressure threshold is tuned to the suction pressure threshold in a stable state, which refers to the corresponding suction pressure threshold when the intracavity pressure recorded by the host controller reaches the pressure control value interval.

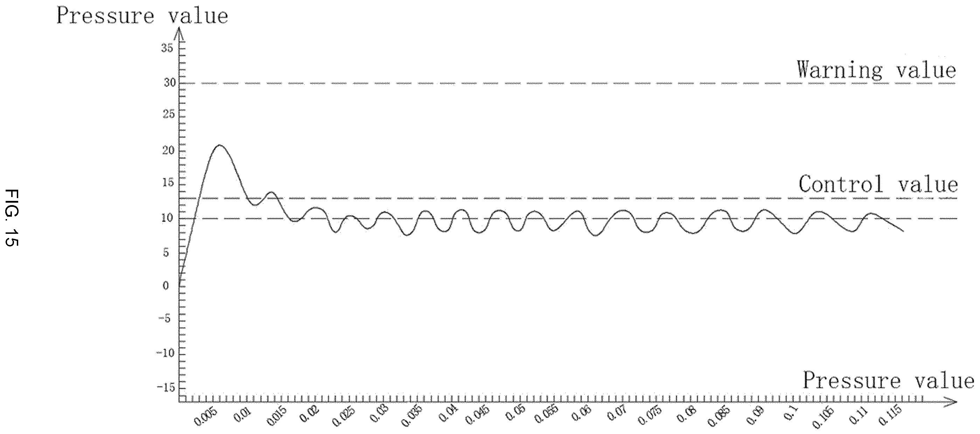

As shown in FIG. 15, when the detected pressure difference exceeds ±3 mmHg and is within ±8 mmHg, indicating that the pressure difference in the cavity is not large. In order to maintain the perfusion demand during operation and keep the perfusion flow rate unchanged, a dynamic balanced perfusion is achieved by controlling the suction pressure threshold, and the intracavity pressure is controlled at the "pressure control value". In this state, there is no need to adjust the perfusion or open the relief valve. This is because the two tuning modes will cause large fluctuations in data. It is also easy to upset the current dynamic balance. Therefore, at this stage, it is necessary to start the fine tuning mode of the system, maintaining the perfusion gear to maintain operation and keeping the relief valve closed, and performing pressure adjustment by finely tuning the suction pressure threshold. In addition, in the process of perfusion and suction, there are many tubelines in the cavity, the pressure fluctuation is large, and the frequency of pressure detection is fast. The setting of a suction pressure threshold also needs to be adjusted in real time according to the change trend of data. In an embodiment of the present disclosure, when the pressure difference exceeds -3 mmHg and is within -8 mmHg, keep the perfusion flow gear unchanged, keep the relief valve closed, maintain the current operation of perfusion, observe the change trend of uploaded data, increase the suction pressure threshold if it is in an ascending stage, decrease the suction pressure threshold if it is in a descending trend, and the reduction amplitude is greater than the amplitude of increasing the suction pressure threshold in the ascending stage; in this way, in the process of data adjustment, the suction pressure threshold tends to be decreased as a whole to achieve the effect of supercharging by fine tuning. When the pressure difference exceeds 3 mmHg and is within 8 mmHg, keep the perfusion operating at the current level; observe the change trend of uploaded data, increase the "suction pressure threshold" if it is in an ascending stage, decrease the "suction pressure threshold" if it is in a descending trend, and the reduction amplitude is smaller than the amplitude of increasing the suction pressure threshold in the ascending stage. In this way, in the process of data adjustment, the "suction pressure threshold" tends to be increased as a whole to achieve the effect of pressure reduction. Judge the state of the current detected pressure value and the data change trend, adjust the suction pressure threshold to make it increase or decrease, and reduce the damage to body cavity caused by large pressure fluctuation.

In some embodiments of the present disclosure, when it is detected that the pressure difference exceeds ±8 mmHg and is within ±20 mmHg, combine fine tuning of the perfusion flow, fine tuning of the relief valve and fine tuning of the suction pressure threshold to jointly act on the intracavity pressure through jointly tuning the perfusion flow gear, the relief valve and the suction pressure threshold, so as to achieve the effect of supercharging or pressure reduction. Specifically, when the detected pressure difference exceeds 8 mmHg and is within 20 mmHg, reduce the perfusion flow rate, observe the change trend of uploaded data, increase the suction pressure threshold if it is in an ascending stage, and decrease the suction pressure threshold if it is in a descending state, and the reduction amplitude is smaller than the amplitude of increasing the suction pressure threshold in the ascending stage; in this way, in the process of data adjustment, the "suction pressure threshold" tends to be increased as a whole to achieve the effect of pressure reduction; when the pressure difference is detected lower than -8 mmHg and is within -20 mmHg, keep the perfusion flow rate at the current level, open the relief valve in stages, observe the change trend of uploaded data, increase the suction pressure threshold if it is in an ascending stage, decrease the "suction pressure threshold" if it is in a descending trend, and the reduction amplitude is greater than the amplitude of increasing the suction pressure threshold in the ascending stage; in this way, in the process of data adjustment, the "suction pressure threshold" tends to be decreased as a whole to achieve the effect of supercharging. In this embodiment, the amplitude of tuning the suction pressure threshold increases with an increase in the pressure difference between the intracavity pressure and the pressure control value. Thus, a combination of coarse tuning and fine tuning is realized through the amplitude of the suction pressure threshold, so that the intracavity pressure quickly reaches the pressure control value to avoid tissue damage caused by excessive pressure fluctuations in the body.

For example, the relationship between the negative pressure suction flow rate in ml/min corresponding to the suction pressure threshold value and is statistically analyzed and classified into 12 categories. They are at -5 mmhg, -10 mmhg, -15 mmhg, -20 mmhg, -25 mmhg, -30 mmhg, -35 mmhg, -40 mmhg, -45 mmhg, -50 mmhg, -60 mmhg and -70 mmhg, respectively. This value is generally used as the default initial value of the system when the system is started, which will be changed later with the dynamic balance process. The value of the balance point will be updated to achieve an effect of dynamic balance.

The pressure differences between the intracavity pressure and the pressure control value Pa in mmhg are counted and then classified. There are 13 states of the intracavity pressure: 1: >20, 2: 15<Pa<=20, 3: 10<Pa<=15, 4: 8<Pa<=10, 5: 5<Pa<=8, 6: 3<Pa<=5, 7: -3<Pa<=3, 8: -5 <Pa<=-3 , 9: -8<Pa<=-5, 10: -10<Pa<=-8,11: - 15<Pa<=-10, 12: -20<Pa<=-15 and 13: -20<Pa.

According to the above statistical classification, the newly collected intracavity pressure data is compared with the historical data, so as to quickly achieve the dynamic balance of perfusion and suction by adjusting the perfusion, suction and relief valves according to the change trend of historical data.

According to the pressure difference Pa in mmhg between the intracavity pressure and the pressure control value, this embodiment provides the following processing mode:
1. (Pa>20): the perfusion runs at the current gear-4, increase the suction pressure, and enhance the suction flow rate according to the negative pressure suction flow rate corresponding to the suction pressure threshold recorded, so that the suction flow rate is greater than the perfusion flow rate; observe the change trend of uploaded data. If it continues to rise, adjust the perfusion to the lowest level and the suction pressure threshold to a threshold of balanced state until the current intracavity pressure returns to the "pressure control value" and restore the perfusion flow rate. If it continues to decrease, adjust the perfusion flow rate according to the changing requirements and increase the suction pressure threshold to achieve the effect of pressure reduction;
2. (15<Pa<=20): the perfusion runs at the current gear-3, and observe the change trend of uploaded data. Increase the "suction pressure threshold +5" if it is in an ascending stage, decrease the "suction pressure threshold -2" if it is in a descending trend. In this way, in the process of data adjustment, the "suction pressure threshold" tends to be increased as a whole to achieve the effect of pressure reduction;
3. (10<Pa<=15): The infusion runs at the current gear-2, and observe the change trend of uploaded data. If it is in the rising stage, increase "suction pressure threshold + 5"; if it is in the decreasing trend, decrease "suction pressure threshold - 3". In this way, in the process of data adjustment, the "suction pressure threshold" tends to be increased as a whole to achieve the effect of pressure reduction;
4. (8<Pa<=10): the perfusion runs at the current gear-1, and observe the change trend of uploaded data. Increase the "suction pressure threshold +4" if it is in an ascending stage, decrease the "suction pressure threshold -1" if it is in a descending trend. In this way, in the process of data adjustment, the "suction pressure threshold" tends to be increased as a whole to achieve the effect of pressure reduction;
5. (5<Pa<=8): maintain the current operation of perfusion, and observe the change trend of uploaded data. Increase the "suction pressure threshold +3" if it is in an ascending stage, decrease the "suction pressure threshold -1" if it is in a descending trend. In this way, in the process of data adjustment, the "suction pressure threshold" tends to be increased as a whole to achieve the effect of pressure reduction;
6. (3<Pa<=5): observe the change trend of uploaded data. Increase the "suction pressure threshold +2" if it is in an ascending stage, decrease the "suction pressure threshold -1" if it is in a descending trend. In this way, in the process of data adjustment, the "suction pressure threshold" tends to be increased as a whole to achieve the effect of pressure reduction;
7. (-3<Pa<=3): keep the current state, start timing and record data. When a certain time is reached, record the current suction pressure threshold (i.e. the suction pressure threshold corresponding to the stable state) and update it in a real-time manner. When the state is destroyed, it shall be re-timed, and the changed saved value can be used for quick recovery of balance during a dynamic adjustment;
8. (-5 <Pa<=-3): maintain the current operation of perfusion, and observe the change trend of uploaded data. Increase the "suction pressure threshold +1" if it is in an ascending stage, decrease the "suction pressure threshold -2" if it is in a descending trend. In this way, in the process of data adjustment, the "suction pressure threshold" tends to be decreased as a whole to achieve the effect of supercharging;
9. (-8<Pa<=-5): maintain the current operation of perfusion, and observe the change trend of uploaded data. Increase the "suction pressure threshold +1" if it is in an ascending stage, decrease the "suction pressure threshold -3" if it is in a descending trend. In this way, in the process of data adjustment, the "suction pressure threshold" tends to be decreased as a whole to achieve the effect of supercharging;
10. (-10<Pa<=-8): maintain the current operation of perfusion, open the relief valve in stages, and observe the change trend of uploaded data. Increase the "suction pressure threshold +1" if it is in an ascending stage, decrease the "suction pressure threshold -4" if it is in a descending trend. In this way, in the process of data adjustment, the "suction pressure threshold" tends to be decreased as a whole to achieve the effect of supercharging;
11. (-15<Pa<=-10): maintain the current operation of perfusion, open the relief valve in stages, and observe the change trend of uploaded data. Increase the "suction pressure threshold +3" if it is in an ascending stage, decrease the "suction pressure threshold -5" if it is in a descending trend. The "suction pressure threshold" tends to be decreased as a whole to achieve the effect of supercharging;
12. (-20<Pa<=-15): the perfusion runs at the current gear+3, open the relief valve and adjust the "suction pressure threshold" to a threshold in a stable state. Observe the change trend of uploaded data. Increase the "suction pressure threshold +2" if it is in an ascending stage, decrease the "suction pressure threshold -5" if it is in a descending trend. In this way, in the process of data adjustment, the "suction pressure threshold" tends to be decreased as a whole to achieve the effect of supercharging;
13. (Pa<-20): the perfusion runs at the current gear+4, open the relief valve and adjust the "suction pressure threshold" to a threshold in a stable state to achieve a rapid balance effect, so that the equipment can be free from the current state as soon as possible.

As above, through the analysis of historical data, the pressure value in the cavity, the pressure control value and the pressure threshold are analyzed to dynamically adjust the perfusion flow rate and the suction flow rate so as to achieve a balance of the intracavity pressure.

In the description of the specification, descriptions with reference to the terms "one embodiment", "some embodiments", "specific embodiments", "optional embodiments", "example", "specific example", or "some examples" mean that specific features, structures, materials or characteristics described in combination with the embodiment or example are contained in at least one embodiment or example of the present disclosure. In the specification, the schematic expressions of the above terms do not necessarily refer to the same embodiments or examples. Moreover, the specific features, structures, materials, or characteristics described may be combined in any one or more embodiments or examples in a suitable manner.

Although embodiments of the present disclosure have been shown and described, the person of ordinary skill in the art can understand that various changes, modifications, substitutions and variations may be made to these embodiments without departing from the principle and purpose of the present disclosure, and the scope of the present disclosure is defined by the claims and their equivalents.

## Claims

1. A sheath tube with pressure measuring function, comprising;
a sheath tube body, having internally a main channel extending from a proximal end to a distal end and a pressure measuring channel, and an interface being provided at a proximal end of the pressure measuring channel;
a pressure detection device, comprising:
a housing, wherein the housing has a piezometric cavity and a mounting cavity, the piezometric cavity has an air inlet for communicating with the interface and a vent hole for communicating with an outside, and a switch is arranged at the vent hole;
a pressure detection assembly, wherein the pressure detection assembly is arranged in the mounting cavity and comprises a gauge pressure sensor, a voltage regulating circuit, an operational amplifier circuit and a conversion circuit; the gauge pressure sensor, the voltage regulating circuit and the operational amplifier circuit are electrically connected with the conversion circuit, and the gauge pressure sensor is used for converting a pressure signal in the piezometric cavity into a voltage signal;
the voltage regulating circuit is configured to increase a voltage difference of the voltage signal, and the operational amplifier circuit multiplies the voltage difference which is increased; the conversion circuit is used for converting the voltage signal which is multiplied into a pressure value; and
an aerogel insulator, and the aerogel insulator is used for transmitting a pressure of the piezometric cavity to the pressure detection assembly.

2. The sheath tube according to claim 1, wherein the gauge pressure sensor has an input voltage positive electrode (+vin), an input voltage negative electrode GND (-vin), an analog signal output voltage positive electrode (+out) and an analog signal output voltage negative electrode (-out); the voltage regulating circuit comprises at least a first resistor (R1) and a second resistor (R3); the first resistor (R1) is connected in parallel between the input voltage positive electrode (+vin) and the analog signal output voltage positive electrode (+out), and the second resistor (R3) is connected in parallel between the input voltage negative electrode GND (-vin) and the analog signal output voltage negative electrode (-out).

3. The sheath tube according to claim 1, wherein the operational amplifier circuit is a GS8332 precision operational amplifier chip, which amplifies the voltage difference by 100 times.

4. The sheath tube according to claim 1, wherein resistance values of the first resistor (R1) and the second resistor (R3) are 100K..

5. The sheath tube according to claim 1, wherein the conversion circuit is a single chip microcomputer stm32f103c8t6.

6. The sheath tube according to claim 1, wherein the housing comprises:
a pedestal, wherein a piezometric tube for forming the piezometric cavity is arranged on the pedestal, the air inlet is arranged at one end of the piezometric tube and the vent hole is formed at an other end of the piezometric tube, and an outlet is arranged on the piezometric tube for outflow of gas in the piezometric tube;
an upper cover disposed above the pedestal;
a PCB board located inside the pedestal and fixed on the upper cover, the PCB board integrates the pressure detection assembly and a control circuit, and the pressure detection assembly is provided just at the outlet and sealed by an aerogel insulator;
a control button disposed on the housing and communicatively connected with the control circuit; and
a connection cable connected with the conversion circuit to output a pressure test value.

7. A detection method of the sheath tube according to any one of claims 1 to 6, comprising:
step 1) conversion stage: the gauge pressure sensor converting the pressure signal into µV-level voltage;
step 2) voltage difference increasing stage: a first resistor (R1) connected in parallel between an input voltage positive electrode (+vin) and an analog signal output voltage positive electrode (+out), and a second resistor (R3) connected in parallel between an input voltage negative electrode GND (-vin) and an analog signal output voltage negative electrode (-out), so as to increase the voltage difference, reach mv level and meet a minimum voltage difference requirement of the operational amplifier circuit;
step 3) operational amplifier stage: amplifying the voltage difference by 100 times through the operational amplifier circuit to reach V (volt) level in a range (0.5-3.0V);
step 4) conversion stage: the voltage difference in step 3) converted into the pressure value by the conversion circuit for output; and
step 5) acquisition stage: the gauge pressure sensor collecting for many times, and a single chip microcomputer removing extreme values to calculate for an average and converts it into the pressure value for output.

8. A pressure detection device, wherein the pressure detection device is the pressure detection device according to any one of claims 1 to 6, comprising;
A housing. The housing has a pressure measuring cavity and a mounting cavity. The pressure measuring cavity has an air inlet for communicating with the pressure measuring interface of the sheath tube and a vent hole for communicating with the outside. A switch is provided at the vent hole.
A pressure detection assembly. The pressure detection assembly is arranged in the mounting cavity and includes a gauge pressure sensor, a voltage adjustment circuit, an operational amplifier circuit, and a conversion circuit. The gauge pressure sensor, the voltage adjustment circuit, the operational amplifier circuit, and the conversion circuit are electrically connected. The gauge pressure sensor is used to convert the pressure signal in the pressure measuring cavity into a voltage signal.
The voltage adjustment circuit is used to increase the voltage difference of the voltage signal. The operational amplifier circuit multiplies the increased voltage difference. The conversion circuit is used to convert the multiplied voltage signal into a pressure value.
an aerogel insulator, which is used for transmitting a pressure of the piezometric cavity to the pressure detection assembly
Preferably, the gauge pressure sensor has an input voltage positive electrode (+vin), an input voltage negative electrode GND (-vin), an analog signal output voltage positive electrode (+out), and an analog signal output voltage negative electrode (-out). The voltage adjustment circuit includes at least a first resistor (R1) and a second resistor (R3). The first resistor (R1) is connected in parallel between the input voltage positive electrode (+vin) and the analog signal output voltage positive electrode (+out), and the second resistor (R3) is connected in parallel between GND (-vin) and the analog signal output voltage negative electrode (-out).
Further preferably, the housing includes:
A base. A pressure measuring tube for forming the pressure measuring cavity is provided on the base. An air inlet is provided at one end of the pressure measuring tube, and a vent hole is formed at the other end. An outlet is provided on the pressure measuring tube for the outflow of gas in the pressure measuring tube.
An upper cover, which is covered above the base.
A PCB board, which is located inside the base and fixed on the upper cover. The PCB board integrates the pressure detection assembly and the control circuit. The pressure detection assembly is arranged directly opposite to the outlet and sealed by an aerogel insulating member.
A control button, which is provided on the housing and is in communication connection with the control circuit.
A connection cable, which is connected to the conversion circuit to output the pressure test value..

9. An intelligent constant pressure regulating perfusion and suction system, comprising
the sheath tube according to any one of claims 1 to 6, wherein the sheath tube body further has a suction channel;
an endoscope inserted in a sheath tube, the endoscope having a liquid delivery channel formed therein;
a perfusion unit communicating with the liquid delivery channel for injecting perfusion solution into a body cavity;
a suction unit communicating with the suction channel for pumping out liquid in the body cavity, the perfusion unit cooperating with the suction unit to maintain the body cavity at an appropriate pressure; and
a host controller communicatively connected with the conversion circuit, the perfusion unit and the suction unit, wherein the host controller controls flow rates and pressures of the perfusion unit and the suction unit according to the pressure value output by the conversion circuit.

10. The constant pressure regulation method of the perfusion and aspiration system according to claim 9, comprising
Step 1) Preset the highest warning pressure value, the lowest warning pressure value, the pressure control value, and the perfusion flow rate gear.
Step 2) The main control unit controls the delivery of the perfusion solution by the perfusion device and the aspiration by the aspiration device, and collects the pressure in the cavity and the negative pressure aspiration pressure.
Step 3) The pressure detection device collects the pressure in the cavity and transmits it to the main control unit. The main control unit automatically adjusts the perfusion and aspiration states according to the real-time monitored pressure value and the data change trend of the pressure value, so that the current pressure in the cavity is balanced at the pressure control value, and the balanced state of perfusion and aspiration is achieved. The perfusion parameters include the perfusion flow rate gear, and the aspiration parameters include the opening of the pressure relief valve and the aspiration pressure threshold.
The pressure regulation modes of the main control unit include a coarse adjustment mode, a fine adjustment mode, and a mixed adjustment mode. The coarse adjustment mode is for the extreme situation of the pressure in the cavity. The fine adjustment mode is for the situation where the pressure difference between the pressure in the cavity and the pressure control value is small. The mixed adjustment mode is for the situation where the pressure in the cavity has not reached the extreme situation and the deviation value is large.
When the pressure difference between the pressure in the cavity and the pressure control value exceeds ±20 mmHg, the coarse adjustment mode is started. When the pressure difference exceeds ±3 mmHg and is within ±8 mmHg, the fine adjustment mode is started. When the detected pressure difference exceeds ±8 mmHg and is within ±20 mmHg, the mixed adjustment mode is started. The coarse adjustment mode includes the following steps. When the pressure value in the body cavity exceeds the highest warning line, the main control unit controls to reduce the perfusion flow rate gear, controls the aspiration pressure threshold so that the aspiration flow rate is greater than the perfusion flow rate. When the pressure difference in the cavity exceeds the lowest warning line, the perfusion flow rate gear will be increased, the pressure relief valve will be opened, and the aspiration pressure threshold will be adjusted to the threshold in the stable state to achieve a rapid balance effect and make the pressure in the cavity get out of the extreme state as soon as possible.
The fine adjustment mode includes the following steps: Keep the perfusion gear running and keep the pressure relief valve closed, and adjust the pressure by finely adjusting the aspiration pressure threshold.
When the pressure difference exceeds -3 mmHg and is within -8 mmHg, keep the perfusion flow rate gear unchanged, keep the pressure relief valve closed, keep the perfusion running at the current level, observe the change trend of the uploaded data. If it is in the rising stage, increase the aspiration pressure threshold. If it is in the falling trend, decrease the aspiration pressure threshold, and the decreasing range is greater than the increasing range of the aspiration pressure threshold in the rising stage. In this way, in the process of data adjustment, there is an overall trend of decreasing the aspiration pressure threshold to achieve the pressurization effect by fine adjustment. When the pressure difference exceeds 3 mmHg and is within 8 mmHg, keep the perfusion running at the current gear, observe the change trend of the uploaded data. If it is in the rising stage, increase the aspiration pressure threshold. If it is in the falling trend, decrease the aspiration pressure threshold, and the decreasing range is less than the increasing range of the aspiration pressure threshold in the rising stage. In the process of data adjustment, there is an overall trend of increasing the aspiration pressure threshold to achieve the pressure reduction effect.
The mixed adjustment mode includes the following steps: Combine the fine adjustment of the perfusion flow rate gear, the pressure relief valve, and the aspiration pressure threshold, and combine the fine adjustment of the perfusion flow rate, the fine adjustment of the pressure relief valve, and the fine adjustment of the aspiration pressure threshold to jointly act on the pressure in the cavity to achieve the pressurization or pressure reduction effect. When the detected pressure difference exceeds 8 mmHg and is within 20 mmHg, appropriately reduce the perfusion flow rate, observe the change trend of the uploaded data. If it is in the rising stage, increase the aspiration pressure threshold. If it is in the falling trend, decrease the aspiration pressure threshold, and the decreasing range is less than the increasing range of the aspiration pressure threshold in the rising stage. In this way, in the process of data adjustment, there is an overall trend of increasing the aspiration pressure threshold to achieve the pressure reduction effect. When the detected pressure difference is lower than -8 mmHg and is within -20 mmHg, keep the perfusion flow rate running at the current level, open the pressure relief valve in stages, observe the change trend of the uploaded data. If it is in the rising stage, increase the aspiration pressure threshold. If it is in the falling trend, decrease the aspiration pressure threshold, and the decreasing range is greater than the increasing range of the aspiration pressure threshold in the rising stage. In this way, in the process of data adjustment, there is an overall trend of decreasing the aspiration pressure threshold to achieve the pressurization effect.
